# EUROPEAN PATENT SPECIFICATION

(11) **EP 4 262 936 B1**
(45) Date of publication and mention of the grant of the patent: **07.10.2026**
(21) Application number: 21820269.5
(22) Date of filing: 06.12.2021
(51) Int. Cl.: A61M 5/20, A61M 5/32, A61M 5/178

(54) **A SUB-ASSEMBLY FOR A MEDICAMENT DELIVERY DEVICE**
UNTERANORDNUNG FÜR EINE MEDIKAMENTENABGABEVORRICHTUNG
SOUS-ENSEMBLE POUR UN DISPOSITIF D'ADMINISTRATION DE MÉDICAMENTS

(30) Priority: 15.12.2020 EP 20214030
(43) Date of publication of application: 25.10.2023
(73) Proprietor: SHL Medical AG, 6302 Zug (CH)
(72) Inventor: GAWELL, Elin, 12155 Johanneshov (SE)
(86) International application number: PCT/EP2021/084373
(87) International publication number: WO 2022/128568

(56) References cited:
- WO-A1-2017/089259
- WO-A1-2019/224785
- WO-A1-2020/239844

## Description

### TECHNICAL FIELD

The present disclosure generally relates to a sub-assembly for a medicament delivery device and particularly to a sub-assembly with a cut-out and a protrusion protruding in the cut-out.

### BACKGROUND

Medicament delivery devices such as pen type injectors or safety syringes are generally known for the self-administration of a medicament by patients without formal medical training. As just one example, those patients suffering from diabetes may require repeated injections of insulin. Other patients may require regular injections of other types of medicaments, such as a growth hormone.

Usually, a medicament delivery device comprises a medicament container, e.g. a syringe or a cartridge, and a medicament delivery member, e.g. a needle or a nozzle.

Some medicament delivery devices will have the medicament delivery members pre-preassembled onto the medicament delivery devices, but keep the medicament delivery members away from the contained medicament containers. Examples can be found in WO2017/089259, WO2020/239844, WO 2009/150078 or WO 2011/112136. For those medicament delivery devices, it is commonly designed that when end users remove a front cover of a medicament delivery device, the pre-preassembled medicament delivery member will be attached to the medicament container at the same time.

For properly attaching the medicament delivery member to the medicament container, those medicament delivery devices usually need the end users to correctly follow instructed steps to manipulate the front cover for removal, for example, the end users may need to twist a front cover first then pull the front cover; or the end users may need to push the front cover first then twist the front cover.

However, not every end user reads a use guide or instructions on a package carefully every time before carrying out a medicament delivery operation; and some end users need to perform multiple medicament delivery operations with multiple different medicament delivery devices, and will be easily confused or mix up the medicament delivery device operation instructions between different medicament delivery devices.

An incorrect manipulation of the cap removal may cause the medicament delivery device malfunction or even injury of the end users. Considering this, it has been appreciated that improvements can be made.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially", "radial", "radially", "rotation", "rotational" and "rotationally" refer to a direction generally perpendicular to the longitudinal direction and at least partially extending around the longitudinal direction.

There is hence provided a sub-assembly for a medicament delivery device, the sub-assembly comprising: a housing extending along a longitudinal axis from a proximal end to a distal end; a cap releasably attached to the proximal end of the housing; one of the cap and the housing comprises a user-visible surface extending in a circumferential direction relative to the axis, the surface comprising a cut-out; the other one of the cap and the housing comprises a protrusion in the cut-out; and the cut-out defines a user-visible labyrinth which the protrusion must follow during use in order for the cap to be detached from the housing.

The sub-assembly provided by this invention can therefore provide a structural arrangement and also a user-visible indication to guide the users for manipulating the medicament delivery device properly.

According to one embodiment, the labyrinth comprises a first longitudinal portion extending along the longitudinal axis between a first end and a second end; and the protrusion is axially movable relative to the user-visible surface between the first end of the first longitudinal portion and the second end of the first longitudinal portion.

According to one embodiment, the labyrinth comprises a first circumferential portion extending around the longitudinal axis between a first end and a second end; and the protrusion is circumferentially movable relative to the user-visible surface between the first end of the first circumferential portion and the second end of the first circumferential portion.

According to one embodiment, the labyrinth comprises a helical portion extending in a direction parallel to the longitudinal axis and in a direction around the longitudinal axis between a first end and a second end; and the protrusion is movable relative to the user-visible surface between the first end of the helical portion and the second end of the helical portion.

According to one embodiment, the helical portion of the labyrinth is connected to the first longitudinal portion of the labyrinth.

According to one embodiment, the helical portion of the labyrinth is connected to the first circumferential portion of the labyrinth.

According to one embodiment, the helical portion of the labyrinth is connected to both the first longitudinal portion of the labyrinth and the first circumferential portion of the labyrinth.

According to one embodiment, the labyrinth comprises a second longitudinal portion extending along the longitudinal axis between a first end and a second end; and the protrusion is axially movable relative to the user-visible surface between the first end of the second longitudinal portion and the second end of the second longitudinal portion.

According to one embodiment, the labyrinth comprises a second circumferential portion extending around the longitudinal axis between a first end and a second end; and the protrusion is circumferentially movable relative to the user-visible surface between the first end of the second circumferential portion and the second end of the second circumferential portion.

According to one embodiment, the sub-assembly comprises a bump extending from an edge of the cut-out of the user-visible surface into the labyrinth.

According to one embodiment, the bump extends in a direction generally transverse to the longitudinal axis into the first longitudinal portion of the labyrinth.

According to one embodiment, the bump extends in the direction of the longitudinal axis into the first circumferential portion of the labyrinth.

According to one embodiment, the bump extends in the direction generally transverse to the longitudinal axis into the second longitudinal portion of the labyrinth.

According to one embodiment, the bump extends in the direction of the longitudinal axis into the second circumferential portion of the labyrinth.

According to one embodiment, the bump extends in the direction of the longitudinal axis into the helical portion of the labyrinth.

According to one embodiment, the labyrinth comprises an exit portion where the protrusion is able to leave the labyrinth by moving past the exit portion.

According to one embodiment, the labyrinth comprises a bridge across a portion of the cut-out, the exit portion is defined by the location of the bridge.

According to one embodiment, the bridge protrudes further from the longitudinal axis than the user-visible surface adjacent to the labyrinth and than the protrusion; thereby allowing the protrusion to pass the bridge to exit the labyrinth, allowing the cap to be detached from the housing.

According to one embodiment, the exit portion of the labyrinth is formed by a flexible wall at least partially on an edge of the cut-out.

According to one embodiment, the exit portion of the labyrinth is a ruptured portion of the cut-out.

According to one embodiment, the exit portion of the labyrinth is a breakable wall of the cut-out.

According to one embodiment, the cap comprises an enclosing body defining an interior area; the housing comprises a proximal portion arranged within the interior area of the enclosing body when the cap is attached to the proximal end of the housing; and wherein the user-visible surface is a part of an external surface of the enclosing body of the cap; and the protrusion is protruding away from the proximal portion of the housing relative to the longitudinal axis.

According to one embodiment, the housing comprises an enclosing portion on the proximal end of the housing; the cap comprises a distal portion enclosed by the enclosing portion of the housing when the cap is attached on the proximal end of the housing; and the user-visible surface is a part of an external surface of the enclosing portion of the housing, and the protrusion is protruding away from the distal portion of the cap relative to the longitudinal axis.

According to one embodiment, sub-assembly is used with a medicament delivery device comprises a medicament delivery member and a medicament container.

According to one embodiment, the medicament delivery member is preassembled to the medicament delivery device and spaced apart from the medicament container; the medicament delivery device is configured to be attached to the medicament container by detaching the cap from the housing; and the detaching movement of the cap from the housing is guided by the labyrinth of the sub-assembly.

According to one embodiment, the medicament delivery member is preassembled to the medicament delivery device and comprises a connection member for establishing a fluid communication between the medicament delivery device and the medicament container; the connection member is spaced apart from the medicament container and is configured to be attached to the medicament container by detaching the cap from the housing; and the detaching movement of the cap from the housing is guided by the labyrinth of the sub-assembly.

According to one embodiment, the labyrinth is configured to guide the cap being moved relative to the housing by a path for a bayonet connection between the cap and the housing.

According to one embodiment, the labyrinth is configured to guide the cap being moved relative to the housing by a path for a thread connection between the cap and the housing.

According to one embodiment, the protrusion comprises at least one different color to an edge of the cut-out; the edge of the cut-out forms the labyrinth.

According to one embodiment, the medicament delivery device that comprises the sub-assembly can be an auto-injector, a pen injector, an inhalation device, or a medical sprayer.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

Embodiments of the inventive concept will now be described, by way of example only, with reference to the accompanying drawings, in which:
Fig 1 schematically shows a perspective view of a sub-assembly for a medicament delivery device.
Fig. 2 schematically shows a perspective view of the sub-assembly of Fig.1 when a cap is detached from a housing.
Fig. 3 schematically shows a perspective view of another example of the cap of the sub-assembly of Fig.1.
Fig. 4A-4D schematically show perspective views of the sub-assembly of Fig.1 before and during detachment of the cap from the housing.
Fig. 5A-5F schematically show perspective views of a needle assembly of a medicament delivery device that comprises the sub-assembly of Fig.1.

### DETAILED DESCRIPTION

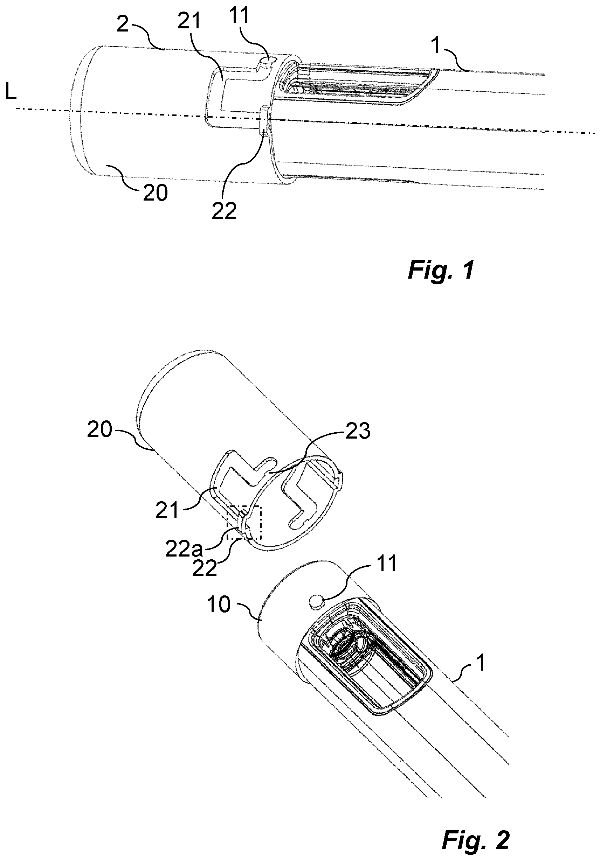

Figs 1-4D illustrate a sub-assembly for a medicament delivery device. The sub-assembly is configured to provide both a visible guide and a structural guide for a user to guide the steps of a manipulation of the medicament delivery device, especially steps for removing a front cover of the medicament delivery device. The front cover of the medicament delivery device is configured to at least partially receive and seal a medicament delivery member, e.g. a needle or a nozzle, of the medicament delivery device.

As shown in Fig. 1, the sub-assembly comprises a housing 1 extending along a longitudinal axis L from a proximal end to a distal end. The housing 1 comprises a generally tubular shaped portion with an interior cavity. The sub-assembly comprises a cap 2 releasably attached to the proximal end of the housing 1. The cap 2 comprises a generally tubular shaped portion with an interior cavity.

The sub-assembly can be an integral part of a medicament delivery device. In this example, the housing 1 is configured to accommodate a medicament container; and the cap 2 is acting as the front cover of the medicament delivery device. The cap 2, in this example is configured to at least partially receive a medicament delivery member that is configured to be attached to the accommodated medicament container while the cap 2 is being removed from the proximal end of the housing 1.

On the other hand, the sub-assembly may be an add-on device for being loaded onto a medicament delivery device. For example, the housing may comprise a distal opening configured to be loaded with a medicament delivery device. The medicament delivery device can be placed into the interior cavity of the housing; or the housing may be formed by two half shells parallel to the longitudinal axis L; the two half shells may be pivotally connected to each other, so that the housing can be pivotally opened and load a medicament delivery device within the interior cavity of the housing. In this example, the cap is configured to be fixed to a front cover of the loaded medicament delivery device, for example, by a connection between a press-fit feature arranged on an inner surface of the generally tubular shaped portion of the cap and the front cover of the loaded medicament delivery device; or a form-fit connection between the interior cavity of the cap and the front cover of the loaded medicament delivery device. In this example, a medicament delivery device can be loaded into the sub-assembly by a care provider, e.g. a doctor, nurse, pharmacist, other caregiver or a medicament delivery device distributor, such as the medicament delivery device distributor, can have the medicament delivery devices loaded before putting the medicament delivery devices into final packages.

The sub-assembly can be used in a medicament delivery device, for example, an auto-injector, a hand-held auto-injector (i.e. the user needs to directly or indirectly, e.g. using an assisting pad to hold, hold the auto-injector during the medicament delivery operation), a pen injector, an inhalation device or a medical sprayer.

One of the cap 2 of the sub-assembly and the housing 1 of the sub-assembly comprises a user-visible surface, the user-visible surface is a surface that is able to be seen by the user from an outside of the sub-assembly when the sub-assembly is assembled to a medicament delivery device. The user-visible surface extends in a circumferential direction relative to the axis, the user-visible surface comprises a cut-out radially arranged relative to the longitudinal axis L; and the other one of the cap 2 of the sub-assembly and the housing 1 of the sub-assembly comprises a protrusion in the cut-out, so that the user is able to see both the cut-out and the protrusion on the user-visible surface directly from the outside of the sub-assembly. The cut-out defines a user-visible labyrinth which the protrusion must follow during use in order for the cap 2 to be detached from the housing 1.

When the protrusion is in the cut-out, the cap 2 cannot be detached from the housing 1 because a moving distance, both along the longitudinal axis and around the longitudinal axis, between the cap 2 and the housing 1 is limited by the labyrinth and the protrusion. Only if the protrusion follows the labyrinth, and finally being moved out from the labyrinth, can the cap 2 be completely detached from the housing 1.

In one embodiment, the cap 2 comprises the cut-out with the labyrinth 21; and the housing 1 comprises the protrusion 11, as shown in Figs 1-4D.

In this embodiment, the cap 2 comprises an enclosing body 20 defining an interior area, the user-visible surface is a part of an external surface of the enclosing body 20, and the housing 1 comprises a proximal portion 10 arranged within the interior area of the enclosing body 20 when the cap 2 is attached to the proximal end of the housing 1. The protrusion 11 is protrudes outwardly relative to the longitudinal axis L from the proximal portion 10 of the housing 1.

The labyrinth 21 comprises an exit portion 22, so if the protrusion 11 is moved, following along the labyrinth 21, and finally arrives at the exit portion 22, the cap 2 can be detached from the housing 1.

In one example, the cap may comprise multiple cut-outs forming labyrinths on the enclosing body rather than a single cut-out; the labyrinths formed by each cut-out are symmetrical relative to one another; for example, the cap may comprise two symmetrically arranged cut-outs forming labyrinths on the enclosing body. In this example, the housing comprises two symmetrical protrusions for matching with the labyrinths. In this example, a force that the user applies to the cap can be evenly distributed on the protrusions and the labyrinths, so that the even if the user is not following the labyrinths and tries to remove the cap from the housing, for example, simply pulling the cap hard relative to the housing, the labyrinths will not be bent since the force is evenly distributed on the protrusions and the labyrinths.

It should be noted that the arrangement of the multiple symmetrically arranged cut-outs with labyrinth and the protrusions is also applied if the housing comprises the cut-outs with labyrinth and the cap comprises the protrusions or a mix of both.

In one embodiment, as shown in Fig. 1, the labyrinth 21 comprises a bridge 22a across a portion of the cut-out, and the exit portion is defined by the location of the bridge 22a. The bridge 22a protrudes further from the longitudinal axis L than the user-visible surface adjacent to the labyrinth; in the example that the user-visible surface is the external surface of the enclosing body 20, as shown in Fig. 2. The part of the bridge 22a that extends across the labyrinth also extends further from the longitudinal axis L than the external surface of the enclosing body 20 adjacent to the labyrinth and then the protrusion 11. This allows the protrusion to pass the bridge to exit the labyrinth, allowing the cap 2 to be detached from the housing 1. The bridge is optional, but can be provided for structural strength.

In another example, the exit portion of the labyrinth is formed by a flexible wall extending at least partially from an edge of the cut-out. For example, the enclosing body 20 of the cap 2 can be formed in more than one material, and the material around the exit portion may be a resilient material, e.g. rubber or silicone. Alternatively, the enclosing body 20 of the cap 2 may be formed of a single material, e.g. plastic, but a thickness (measured along the transverse direction relative to the longitudinal axis L) of the wall is less than a thickness (measured along the transverse direction relative to the longitudinal axis L) of other parts of the enclosing body 20 of the cap 2 around the labyrinth.

The wall of the labyrinth is configured to be deflected radially outward relative to the longitudinal axis L by the protrusion 11 when the protrusion 11 axially moves past, so that the protrusion 11 will then leave the labyrinth once the protrusion 11 passes the exit portion. In a preferred example, the protrusion 11 comprises a distally directed ramp surface, and the flexible wall that forms the exit portion comprises a proximally directed ramp surface, so that when the distally directed ramp surface of the protrusion 11 contacts with the proximally directed ramp surface, the flexible wall may be more easily deflected radially outward.

Furthermore, the flexible wall that forms the exit portion in this example may comprise one or more slots, so that the flexible wall can be more flexible due to the material reduction, and therefore reduce the material tensile force at the same time.

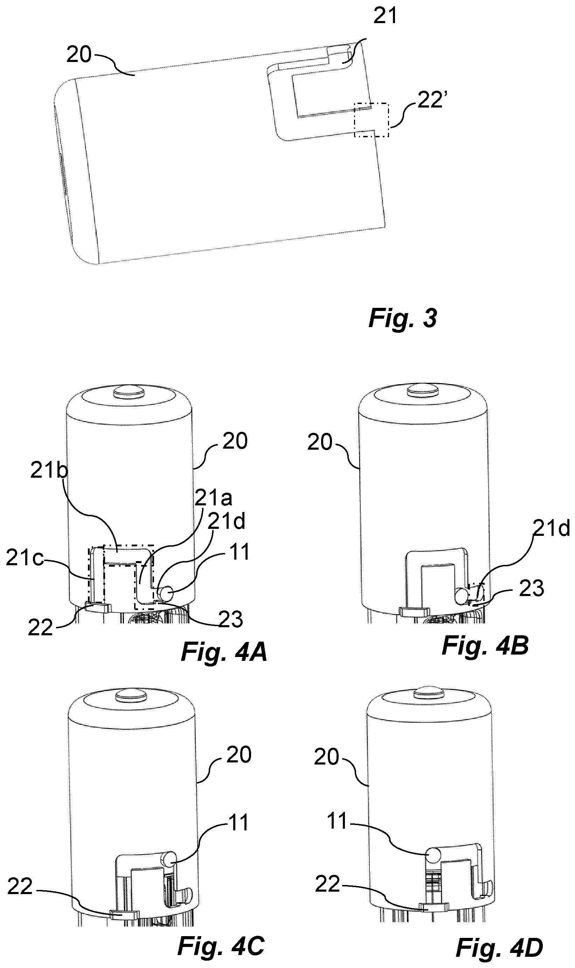

In another embodiment, the exit portion 22' of the labyrinth 21 is a ruptured portion of the cut-out, meaning that at the exit portion 22' of the labyrinth 21, there is no more a wall from the cut-out blocking the longitudinal movement of the protrusion 11, so that the protrusion 11 is able to leave the labyrinth 21 once the protrusion 11 is moved past the exit portion 22' of the labyrinth 21, as shown in Fig. 3.

In another embodiment, the exit portion of the labyrinth is a portion of the cut-out that can rupture (for example a breakable wall or seal extending across the exit portion), meaning that at the exit portion of the labyrinth, that the protrusion is able to leave the labyrinth through the portion of the cut-out that can rupture. In this example, the breakable wall/ seal can be a tamper-evident seal to indicate whether the medicament delivery device is used or is no longer in a sterilized situation.

The exit portion is preferably located on an edge of the cap 2 or the housing 1. In the embodiment shown in Figs 1-3, the exit portion is on a distal end edge of the cap 2.

It should be noted that, alternatively, the cut-out (and also the labyrinth) can be arranged on the housing 1, and the protrusion can be arranged on the cap. For example, the housing may comprise an enclosing portion on the proximal end of the housing; and the cap comprises a distal portion enclosed by the enclosing portion of the housing when the cap is attached on the proximal end of the housing. In this example, the user-visible surface is a part of an external surface of the enclosing portion of the housing, and the protrusion is outwardly protruding from the distal portion of the cap. In this example, the exit portion is preferably arranged on a proximal end edge of the housing.

The labyrinth 21 may comprise one or more longitudinal portions, one or more circumferential portions, and/or one or more helical portions. The connection between the one or more longitudinal portions and/ or the one or more circumferential portions and/ or the one or more helical portions is dependent on the design of the medicament delivery device that will be loaded into.

For example, WO 2009150078 discloses a medicament delivery device with a preassembled medicament delivery member that can be attached to a contained medicament container through a bayonet connection or a thread connection between a medicament delivery member holder and a medicament container adaptor. However, the user needs to manipulate the cap along a path for the bayonet connection or the thread connection, so that the when the end user removes the cap, the medicament delivery device can be also attached to the contained medicament container.

In examples where the user needs to attach the preassembled medicament delivery member to the contained medicament container through a bayonet connection together with the movement of the cap removal, the labyrinth 21 should comprise a first longitudinal portion 21a extending along the longitudinal axis L between a first end and a second end. The labyrinth 21 comprises a first circumferential portion 21b extending around the longitudinal axis L between a first end and a second end. The second end of the first longitudinal portion 21a is connected to the first end of the circumferential portion 21b. The exit portion of the cut-out is located at the second end of the circumferential portion. In such an embodiment, the longitudinal extent of the cap 2 varies at different points around the cap. During assembly on a production line, the protrusion 11 should be initially positioned at the first end of the first longitudinal portion 21a of the labyrinth 21, similar to the position shown in Fig. 4B.

In this example, to detach the cap from the housing, the user can only move the cap and the housing longitudinally relative to each other first, then can only move the cap and the housing circumferentially relative to each other, shown in Fig. 4C-4D, due to the structural limitation between the labyrinth 21 and protrusion 11. When the protrusion reaches the exit portion of the labyrinth, the user is able to detach the cap from the housing.

Alternatively, instead of locating the exit portion at the second end of the first circumferential portion 21b of the labyrinth, the labyrinth comprises a second longitudinal portion 21c extending along the longitudinal axis L between a first end and a second end. The first end of the second longitudinal portion 21c is connected to the second end of the first circumferential portion 21b, and the exit portion 22 is located at the second end of the second longitudinal portion 21c. In this example, the protrusion will be guided to move to the edge of the cap or housing, so that the protrusion can leave the labyrinth more easily.

Furthermore, the labyrinth 21 may comprise a second circumferential portion 21d extending around the longitudinal axis L between a first end and a second end. Instead of assembling the protrusion 11 to be located at the first end of the first longitudinal portion 21a of the labyrinth, the protrusion 11 can be assembled to locate at the first end of the second circumferential portion 21d of the labyrinth 21 during assembly on the production line. The second end of the second circumferential portion 21d of the labyrinth 21 is connected to the first end of the first longitudinal portion 21a of the labyrinth 21, as shown in 4A-4D.

The first longitudinal portion 21a of the cut-out 21 and the first circumferential portion 21b of the cut-out 21 are configured to guide the user to detach the cap from the housing through the bayonet connection path, so that the medicament delivery member can be attached to the medicament container. The second longitudinal portion 21c of the labyrinth 21 and/ or the second circumferential portion 21d of the labyrinth 21 are optional security features. For example, the second circumferential portion 21d of the labyrinth 21 is configured to prevent an unintentional use of the medicament delivery device if the end user accidentally pushes on the cap 2; so that the contained medicament can be retained in a sterilized state until the end user plans to use the medicament delivery device.

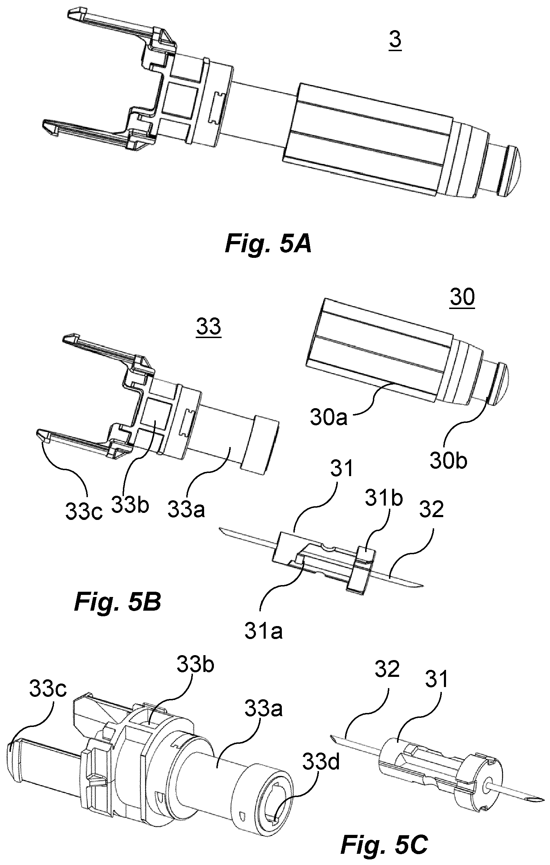

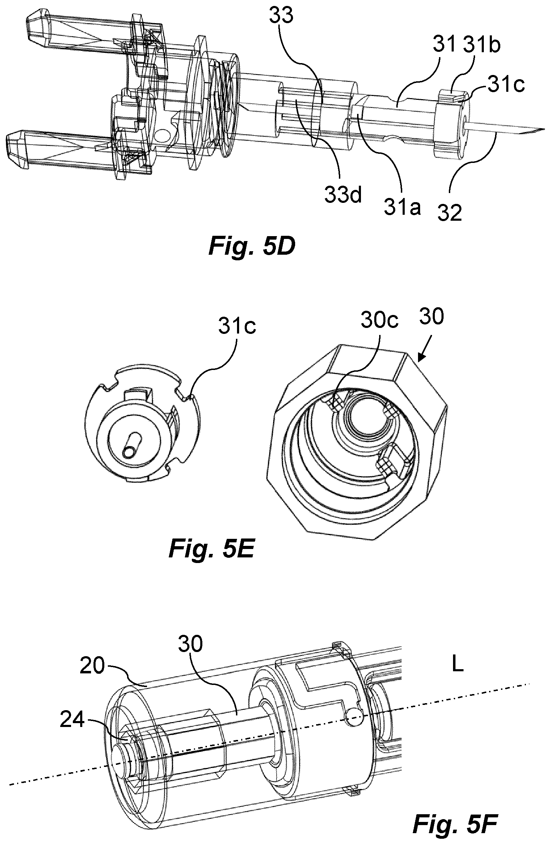

In a preferred example, the sub-assembly of the invention is used in a medicament delivery device that comprises a needle assembly 3, as shown in Figs 5A-5F. The needle assembly is configured to be attached to a medicament container of the medicament delivery device when the user removes the cap 2 from the housing 1. In this example, the needle assembly 3 comprises a needle cap 30, a needle holder 31, a needle 32, and a connector 33. The needle cap 30 comprises a cap body 30a configured to receive a least a part of the needle 32 and a least a part of the needle holder 31. The cap body is attached to the cap 2, so that the cap body 30a is axially and rotationally immovable relative to the cap 2. In a preferred example, the cap body 30a is rotationally engaged with the cap body 20 of the cap 2. In one example, the cap body 30a is hexagon, rectangular or triangle shaped (when observes along the longitudinal axis). Alternatively, the cap body comprises a protrusion protruding from an outer surface of the cap body. In this example, the enclosing body 20 of the cap 2 comprises a fastener 24 formed with either a shape match to the shape of the cap body 30a or a counter protrusion that engages with the protrusion of the cap body. Furthermore, the cap body 30a is axially engaged with the enclosing body 20 of the cap 2. In this example, an opening is arranged in a wall of the fastener 24 of the cap 2, a flange 30b of the cap body 30a engages with a proximally directed surface of the fastener 24 of the cap 2, as shown in Fig. 5F.

In this example, the needle 32 is attached to the needle holder 31, e.g. by glue. The needle holder 31 comprises a rib 31a protruding from an outer surface of the needle holder 31, a flange section 31b, and a cut-out 31c. The connector 33 comprises a first tubular section 33a for receiving at least a part of the needle holder 31, a second tubular section 33b for receiving a proximal end of the medicament container of the medicament delivery device, and a fastener 33c for attaching the needle assembly 3 to the housing 1 of the medicament delivery device. In a preferred example, the fastener 33c is a snap-fit arm.

In this example, the connector 33 comprises a slot 33d in a wall of the first tubular section 33a. In a preferred example, the slot 33d opens towards a central axis of the first tubular section 33a. The rib 31a of the needle holder 31 is configured to be situated at a proximal edge of the first tubular section 33a of the connector 33 in an original position. When the user plans to use the medicament delivery device, the needle holder 31 is configured to be rotated by the needle cap 30 (by the engagement between the cut-out 31c of the needle holder 31 and a rib 30c of the needle cap, as shown in Fig. 5E) around the longitudinal axis relative to the connector 33 to be aligned with the slot 33d in the direction of the longitudinal axis L. The needle holder 31 is then configured to be moved along the slot 33d relative to the connector 33 in the distal direction for establishing a fluid communication between the needle 32 and the medicament container, preferably by an engagement between the rib 30c of the needle cap 30 and flange section 31b of the needle holder 31. For example, the rib 30c comprises a first section for engaging with the cut-out 31c and a second section for engaging with a proximally directed surface of the flange section 31b. In a preferred example, once a distal end of the needle holder 31 arrives at a distal end of the slot 33d, the user should rotate the needle holder 31 around the longitudinal axis L relative to the connector 33, so that the rib 31a is offset relative to the slot 33d of the connector 33 in the direction of the longitudinal axis L. Therefore, the rib 31a is blocked by the wall of the connector 33 in the proximal direction relative to the connector 33.

In this example, the labyrinth 21 the first longitudinal portion 21a, the first circumferential portion 21b, the second longitudinal portion 21c, and the second circumferential portion 21d, as shown in Figs 4A-4D. The user will be guided by the labyrinth 21 and the protrusion 11, to perform the following actions with the following order, since the needle cap 30 is engaged with the enclosing body 20 and the needle holder 31, the needle holder 31 will have the same movement as the enclosing body of the cap 2.
1. Rotating the enclosing body 20 of the cap 2 relative to the housing 1 around the longitudinal axis L. The needle holder 31 is also rotated so that the rib 31a is aligned with the slot 33d of the connector 33 in the direction of the longitudinal axis L.
2. Pushing the enclosing body 20 of the cap 2 relative to the housing 1 towards the distal direction of the housing 1 in the direction of the longitudinal axis L. The needle holder 31 is also pushed in the distal direction relative to the connector 33, so that the rib 31a is moved along the slot 33d of the connector 33.
3. Rotating the enclosing body 20 of the cap 2 relative to the housing 1 around the longitudinal axis L. The needle holder 31 is also rotated so that the rib 31a is offset relative to the slot 33d of the connector 33 in the direction of the longitudinal axis L.
4. Pulling the enclosing body 20 of the cap 2 relative to the housing 1 towards the proximal direction of the housing 1 in the direction of the longitudinal axis L. Since the rib 30c of the needle cap 30 is engaged the proximally directed surface of the flange section 31b of the needle holder 3, when the enclosing body 20 of the cap 2 is moved relative to the housing 1 towards the proximal direction of the housing 1 in the direction of the longitudinal axis L, the needle cap 30 will move together with the enclosing body 20 of the cap 2 and detach from the housing 1.

The user's behavior of manipulating the cap 2 removal from the housing 1 is not only structurally guided by the labyrinth 21 and the protrusion 11, but also visually guided by the labyrinth 21 and the protrusion 11, since both the cut-out 21 and the protrusion 11 can preferably be observed from the outside of the medicament delivery device. In a preferred example, the protrusion 11 may comprise a bright or eye-catching color, e.g. red, yellow; or a contrasting color relative to the enclosing body 20 of the cap 2 when the labyrinth 21 is arranged on the cap 2; or the housing when the labyrinth is arranged on the housing; so that the user can easily notice the protrusion 11, and easily understand when the cap 2 can be fully detached from the housing 1.

In another example that the end user needs to attach the preassembled medicament delivery member to the contained medicament container through a thread connection together with a movement of cap removal, the labyrinth should comprise a helical portion longitudinally and circumferentially extending from a first end to a second end. During assembly on a production line, the protrusion should be initially positioned at the first end of the helical portion of the labyrinth. The exit portion is located at the second end of the helical portion of the labyrinth, and only when the end user twists the cap relative to the housing along the helical portion of the labyrinth is the end user able to detach the cap from the housing. As described above, it is also possible in this example to include the second longitudinal portion and/ or the second circumferential portion in the labyrinth as one or more security features.

In another example where the end user needs to attach the preassembled medicament delivery member to the contained medicament container through a snap-fit connection, the user may need to remove the cap from the housing by pushing the cap relative to the housing first, then pulling the cap out from the housing. In this example, the labyrinth may comprise a first longitudinal portion for guiding the end user to push the cap relative to the housing, and a second longitudinal portion for guiding the end user to pull the cap relative to the housing. The first longitudinal portion and the second longitudinal portion are connected through a helical portion, the labyrinth in this example may be therefore formed generally as a "V" shape on the enclosing body of the cap/ or on the enclosing portion of the housing.

Besides the one or more longitudinal portions, one or more circumferential portions, and/or one or more helical portions of the labyrinth, the sub-assembly may comprise a bump 23 extending from an edge of the cut-out of the user-visible surface into the labyrinth. Preferably, the bump 23 is located in the portion of the labyrinth 21 where the protrusion 11 is located immediately after assembly. For example, if the protrusion 11 is at the first end of the second circumferential portion 21d of the labyrinth 21 after assembly on the production line, then the bump 23 can be located in the second circumferential portion 21d, as shown in Fig. 4A. In this case, the bump 23 will extend in the direction of the longitudinal axis L into the second circumferential portion 21d. The bump 23 is configured to provide the end user with a tactile indication that the sterilized state of the contained medicament will break and/or to provide initial resistance to the movement of the protrusion in the labyrinth to make it harder to accidentally initiate cap removal.

It should be noted that the one or more longitudinal portions, one or more circumferential portions, and/or one or more helical portions of the labyrinth can be partially formed with another shapes, e.g. arrow shapes. For example, if the front cover of the medicament delivery device can be detached by twisting 180 degree clockwise around the longitudinal axis L relative to the housing, the labyrinth optionally only comprises one circumferential portion extending around be longitudinal axis L between a first end and a second end. The second end may be formed as an arrow shape pointing toward the clockwise direction, and the exit portion may be located at the second end of the circumferential portion.

It should be noted that example mechanisms for detaching a front cover from a medicament delivery device and attaching a preassembled medicament delivery member to a medicament container in a medicament delivery device by removing the front cover are described in documents such as WO2009/150078 and WO2011/112136.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device, the sub-assembly comprising:
a housing (1) extending along a longitudinal axis (L) from a proximal end to a distal end;
a cap (2) releasably attached to the proximal end of the housing (1);
wherein one of the cap (2) and the housing (1) comprises a user-visible surface extending in a circumferential direction relative to the axis, the surface comprising a cut-out;
a needle assembly (3); wherein the needle assembly (3) comprises a needle (32) and a needle cap (30) at least partially enclosing the needle (32); and wherein the needle cap (30) is attached to the cap(2);
wherein the other one of the cap (2) and the housing (1) comprises a protrusion (11) in the cut-out; and
wherein the cut-out defines a user-visible labyrinth (21) which the protrusion (11) must follow during use in order for the cap (2) to be detached from the housing (1); wherein the labyrinth (21) comprises a first circumferential portion (21b) extending around the longitudinal axis (L) between a first end and a second end; and wherein the protrusion (11) is circumferentially movable relative to the user-visible surface between the first end of the first circumferential portion (21b) and the second end of the first circumferential portion (21b); and
**characterized in that** the needle assembly further comprises a needle holder (31) attached to the needle (32) and a connector (33) comprising a first tubular section (33a) for receiving at least a part of the needle holder (31);
wherein the needle cap (30) comprises a rib (30c) partially situated within a cut-out of the needle holder (31) and partially engaged with a proximally directed surface of the needle holder (31); wherein the connector (33) comprises a slot (33d) in a wall of the first tubular section (33a); wherein the needle holder (31) comprises a rib (31a) protruding from an outer surface of the needle holder (31); wherein
the rib (31a) of the needle holder (31) is configured to be aligned with the slot (33d) of the connector (33) in the direction of the longitudinal axis (L) when a user of the medicament delivery device rotates the needle holder (31) around the longitudinal axis (L) by rotating the needle cap (30) and the cap (2).

2. The medicament delivery device as claimed in any one of the preceding claims, wherein the labyrinth (21) comprises a first longitudinal portion (21a) extending along the longitudinal axis (L) between a first end and a second end; and wherein the protrusion (11) is axially movable relative to the user-visible surface between the first end of the first longitudinal portion (21a) and the second end of the first longitudinal portion (21a).

3. The medicament delivery device as claimed in claim 2, wherein the labyrinth (21) comprises a second longitudinal portion (21c) extending along the longitudinal axis (L) between a first end and a second end; and wherein the protrusion (11) is axially movable relative to the user-visible surface between the first end of the second longitudinal portion (21c) and the second end of the second longitudinal portion (21c).

4. The medicament delivery device as claimed in claim 3, wherein the labyrinth (21) comprises a second circumferential portion (21d) extending around the longitudinal axis (L) between a first end and a second end; and wherein the protrusion (11) is circumferentially movable relative to the user-visible surface between the first end of the second circumferential portion (21d) and the second end of the second circumferential portion (21d).

5. The medicament delivery device as claimed in any one of the preceding claims, wherein the sub-assembly comprises a bump (23) extending from an edge of the cut-out of the user-visible surface into the labyrinth (21).

6. The medicament delivery device as claimed in claim 5, when dependent on claim 4, wherein the bump (23) extends in the direction of the longitudinal axis (L) into the second circumferential portion (21d) of the labyrinth (21).

7. The medicament delivery device of any one of preceding claims, wherein the medicament delivery device is an auto-injector.

## Patentansprüche

1. Arzneimittelabgabevorrichtung, die Unteranordnung umfassend:
ein Gehäuse (1), das sich entlang einer Längsachse (L) von einem proximalen Ende zu einem distalen Ende erstreckt;
eine Kappe (2), die an dem proximalen Ende des Gehäuses (1) lösbar angebracht ist;
wobei eines von der Kappe (2) und dem Gehäuse (1) eine für einen Nutzer sichtbare Oberfläche, die sich in eine Umfangsrichtung relativ zu der Achse erstreckt, umfasst, die Oberfläche umfassend eine Ausschneidung;
eine Nadelanordnung (3); wobei die Nadelanordnung (3) eine Nadel (32) und eine Nadelkappe (30), die die Nadel (32) mindestens teilweise umschließt, umfasst; und wobei die Nadelkappe (30) an der Kappe (2) angebracht ist;
wobei das andere von der Kappe (2) und dem Gehäuse (1) einen Vorsprung (11) in der Ausschneidung umfasst; und
wobei die Ausschneidung ein für den Nutzer sichtbares Labyrinth (21) definiert, dem der Vorsprung (11) während einer Verwendung folgen muss, damit die Kappe (2) von dem Gehäuse (1) gelöst wird; wobei das Labyrinth (21) einen ersten Umfangsabschnitt (21b), der sich um die Längsachse (L) zwischen einem ersten Ende und einem zweiten Ende erstreckt, umfasst; und wobei der Vorsprung (11) relativ zu der für den Nutzer sichtbaren Oberfläche zwischen dem ersten Ende des ersten Umfangsabschnitts (21b) und dem zweiten Ende des ersten Umfangsabschnitts (21b) umfänglich bewegbar ist; und
**dadurch gekennzeichnet, dass** die Nadelanordnung ferner einen Nadelhalter (31), der an der Nadel (32) angebracht ist, und einen Verbinder (33), umfassend einen ersten röhrenförmigen Bereich (33a) zum Aufnehmen mindestens eines Teils des Nadelhalters (31), umfasst; wobei die Nadelkappe (30) eine Rippe (30c), die teilweise innerhalb einer Ausschneidung des Nadelhalters (31) liegt und teilweise mit einer proximal gerichteten Oberfläche des Nadelhalters (31) in Eingriff steht, umfasst; wobei der Verbinder (33) einen Schlitz (33d) in einer Wand des ersten röhrenförmigen Bereichs (33a) umfasst; wobei der Nadelhalter (31) eine Rippe (31a), die von einer Außenoberfläche des Nadelhalters (31) vorspringt, umfasst; wobei
die Rippe (31a) des Nadelhalters (31) konfiguriert ist, um an dem Schlitz (33d) des Verbinders (33) in der Richtung der Längsachse (L) ausgerichtet zu werden, wenn ein Nutzer der Arzneimittelabgabevorrichtung den Nadelhalter (31) durch Drehen der Nadelkappe (30) und der Kappe (2) um die Längsachse (L) dreht.

2. Arzneimittelabgabevorrichtung nach einem der vorstehenden Ansprüche, wobei das Labyrinth (21) einen ersten Längsabschnitt (21a), der sich entlang der Längsachse (L) zwischen einem ersten Ende und einem zweiten Ende erstreckt, umfasst; und wobei der Vorsprung (11) relativ zu der für den Nutzer sichtbaren Oberfläche zwischen dem ersten Ende des ersten Längsabschnitts (21a) und dem zweiten Ende des ersten Längsabschnitts (21a) axial bewegbar ist.

3. Arzneimittelabgabevorrichtung nach Anspruch 2, wobei das Labyrinth (21) einen zweiten Längsabschnitt (21c), der sich entlang der Längsachse (L) zwischen einem ersten Ende und einem zweiten Ende erstreckt, umfasst; und wobei der Vorsprung (11) relativ zu der für den Nutzer sichtbaren Oberfläche zwischen dem ersten Ende des zweiten Längsabschnitts (21c) und dem zweiten Ende des zweiten Längsabschnitts (21c) axial bewegbar ist.

4. Arzneimittelabgabevorrichtung nach Anspruch 3, wobei das Labyrinth (21) einen zweiten Umfangsabschnitt (21d), der sich um die Längsachse (L) zwischen einem ersten Ende und einem zweiten Ende erstreckt, umfasst; und wobei der Vorsprung (11) relativ zu der für den Nutzer sichtbaren Oberfläche zwischen dem ersten Ende des zweiten Umfangsabschnitts (21d) und dem zweiten Ende des zweiten Umfangsabschnitts (21d) umfänglich bewegbar ist.

5. Arzneimittelabgabevorrichtung nach einem der vorstehenden Ansprüche, wobei die Unteranordnung eine Erhebung (23), die sich von einer Kante der Ausschneidung der für den Nutzer sichtbaren Oberfläche in das Labyrinth (21) erstreckt, umfasst.

6. Arzneimittelabgabevorrichtung nach Anspruch 5, wenn abhängig von Anspruch 4, wobei sich die Erhebung (23) in die Richtung der Längsachse (L) in den zweiten Umfangsabschnitt (21d) des Labyrinths (21) erstreckt.

7. Arzneimittelabgabevorrichtung nach einem der vorstehenden Ansprüche, wobei die Arzneimittelabgabevorrichtung ein Autoinjektor ist.

## Revendications

1. Dispositif d'administration de médicament , le sous-ensemble comprenant :
un boîtier (1) s'étendant le long d'un axe longitudinal (L) depuis une extrémité proximale à une extrémité distale ;
un capuchon (2) fixé de manière amovible à l'extrémité proximale du boîtier (1) ;
dans lequel un élément parmi le capuchon (2) et le boîtier (1) comprend une surface visible par l'utilisateur s'étendant dans une direction circonférentielle par rapport à l'axe, la surface comprenant une découpe ;
un ensemble aiguille (3) ; dans lequel l'ensemble aiguille (3) comprend une aiguille (32) et un capuchon d'aiguille (30) entourant au moins partiellement l'aiguille (32) ; et dans lequel le capuchon d'aiguille (30) est fixé au capuchon (2) ;
dans lequel l'autre élément parmi le capuchon (2) et le boîtier (1) comprend une saillie (11) dans la découpe ; et
dans lequel la découpe définit un labyrinthe (21) visible par l'utilisateur que la saillie (11) doit suivre pendant l'utilisation afin que le capuchon (2) se détache du boîtier (1) ; dans lequel le labyrinthe (21) comprend une première partie circonférentielle (21b) s'étendant autour de l'axe longitudinal (L) entre une première extrémité et une seconde extrémité ; et dans lequel la saillie (11) est mobile circonférentiellement par rapport à la surface visible par l'utilisateur entre la première extrémité de la première partie circonférentielle (21b) et la seconde extrémité de la première partie circonférentielle (21b) ; et
**caractérisé en ce que** l'ensemble aiguille comprend en outre un porte-aiguille (31) fixé à l'aiguille (32) et un raccord (33) comprenant une première section tubulaire (33a) destinée à recevoir au moins une partie du porte-aiguille (31) ; dans lequel le capuchon d'aiguille (30) comprend une nervure (30c) partiellement située à l'intérieur d'une découpe du porte-aiguille (31) et partiellement en prise avec une surface dirigée proximalement du porte-aiguille (31) ; dans lequel le raccord (33) comprend une fente (33d) dans une paroi de la première section tubulaire (33a) ; dans lequel le porte-aiguille (31) comprend une nervure (31a) faisant saillie à partir d'une surface externe du porte-aiguille (31) ; dans lequel
la nervure (31a) du porte-aiguille (31) est conçue pour être alignée avec la fente (33d) du raccord (33) dans la direction de l'axe longitudinal (L) lorsqu'un utilisateur du dispositif d'administration de médicament fait tourner le porte-aiguille (31) autour de l'axe longitudinal (L) par la rotation du capuchon d'aiguille (30) et du capuchon (2).

2. Dispositif d'administration de médicament selon l'une quelconque des revendications précédentes, dans lequel le labyrinthe (21) comprend une première partie longitudinale (21a) s'étendant le long de l'axe longitudinal (L) entre une première extrémité et une seconde extrémité ; et dans lequel la saillie (11) est mobile axialement par rapport à la surface visible par l'utilisateur entre la première extrémité de la première partie longitudinale (21a) et la seconde extrémité de la première partie longitudinale (21a).

3. Dispositif d'administration de médicament selon la revendication 2, dans lequel le labyrinthe (21) comprend une seconde partie longitudinale (21c) s'étendant le long de l'axe longitudinal (L) entre une première extrémité et une seconde extrémité ; et dans lequel la saillie (11) est mobile axialement par rapport à la surface visible par l'utilisateur entre la première extrémité de la seconde partie longitudinale (21c) et la seconde extrémité de la seconde partie longitudinale (21c).

4. Dispositif d'administration de médicament selon la revendication 3, dans lequel le labyrinthe (21) comprend une seconde partie circonférentielle (21d) s'étendant autour de l'axe longitudinal (L) entre une première extrémité et une seconde extrémité ; et dans lequel la saillie (11) est mobile circonférentiellement par rapport à la surface visible par l'utilisateur entre la première extrémité de la seconde partie circonférentielle (21d) et la seconde extrémité de la seconde partie circonférentielle (21d).

5. Dispositif d'administration de médicament selon l'une quelconque des revendications précédentes, dans lequel le sous-ensemble comprend une bosse (23) s'étendant à partir d'un bord de la découpe de la surface visible par l'utilisateur dans le labyrinthe (21).

6. Dispositif d'administration de médicament selon la revendication 5, lorsqu'elle dépend de la revendication 4, dans lequel la bosse (23) s'étend dans la direction de l'axe longitudinal (L) dans la seconde partie circonférentielle (21d) du labyrinthe (21).

7. Dispositif d'administration de médicament selon l'une quelconque des revendications précédentes, dans lequel le dispositif d'administration de médicament est un auto-injecteur.
